# EUROPEAN PATENT APPLICATION

(11) **EP 2 003 208 A2**
(43) Date of publication of application: **17.12.2008**
(21) Application number: 07740539.7
(22) Date of filing: 30.03.2007
(51) Int. Cl.: C12P 13/02, C08F 20/56

(54) **METHOD OF PRODUCING ACRYLAMIDE**

(30) Priority: 06.04.2006 JP 2006105464
(71) Applicant: Mitsui Chemicals, Inc., Tokyo 105-7117 (JP)
(72) Inventor: ABE, Takeya MITSUI CHEMICALS, INC., Takaishi-shi Osaka 592-8501 (JP); FUKUDA, Takeshi MITSUI CHEMICALS, INC., Takaishi-shi Osaka 592-8501 (JP); MURAMOTO, Masanori MITSUI CHEMICALS, INC., Takaishi-shi Osaka 592-8501 (JP); HAZAMA, Souichi MITSUI CHEMICALS, INC., Takaishi-shi Osaka 592-8501 (JP)
(74) Representative: Wytenburg, Wilhelmus Johannes
(86) International application number: PCT/JP2007/057103
(87) International publication number: WO 2007/116824

(57) **Abstract**

An object of the present invention is to provide a method for more efficiently producing acrylamide with higher quality by a microbial catalyst and the like containing a nitrile hydratase. According to the present invention, the acrylamide may be produced by hydrating acrylonitrile by a microbial biomass containing a nitrile hydratase or a processed product of the microbial biomass in the presence of at least one compound having an active methylene group and/or at least one salt of the compound.

## Description

### [TECHNICAL FIELD]

The present invention relates to a method for producing acrylamide and more particularly to a method for producing of high quality by effectively hydrating acrylonitrile by a microbial biomass containing a nitrile hydratase, and the like.

### [BACKGROUND ART]

One of the main methods for producing acrylamide includes a method of hydrating acrylonitrile. For example, there is known a method of hydrating acrylonitrile by a metallic copper catalyst such as Raney copper and the like or a method of hydrating acrylonitrile by using as a catalyst a microbial biomass containing a nitrile hydratase or a processed product of the microbial biomass, and the like.

Among these, as an industrial production method, the method for producing acrylamide using the microbial biomass containing a nitrile hydratase, and the like as a catalyst has attracted attention because the method has a high conversion of acrylonitrile and high selectivity, compared to a method of hydration using a conventional metallic copper catalyst and the like.

In order to efficiently produce acrylamides with higher quality by using the microbial biomass containing a nitrile hydratase, and the like as a catalyst, impurities inhibiting the catalytic action of the microbial biomass and the like are required to be removed as much as possible.

In addition, acrylamides obtained by such reactions are mainly used as a raw material for an acrylamide-based polymer. Recently a further improvement of the quality is required for an acrylamide-based polymer. For example, the applications of an acrylamide-based polymer include a flocculant. Recently, the acrylamide-based polymer used as a flocculant is expected to have a higher molecular weight while maintaining the solubility in water associated with requirement for performance improvement. Further, the acrylamide-based polymer is used as an additive for manufacturing paper, and the like, however, as the additive for manufacturing paper, a polymer being more excellent in hue is required in order to further improve the quality of the resulting paper.

As a method for improving the quality of the acrylamide obtained by using a microbial catalyst containing a nitrile hydratase, and the like or the quality of polyacrylamide, there are known a method in which the concentration of hydrocyanic acid in a nitrile compound is reduced by a chemical process and then the nitrile hydratase is acted on the nitrile compound to produce an amide compound (for example, see Patent Document 1), and a method in which oxazole and hydrocyanic acid contained in acrylonitrile as impurities are reduced and then the acrylonitrile is converted to acrylamide, followed by producing an acrylamide-based polymer from the acrylamide (for example, see Patent Document 2), and the like.

However, it is difficult to completely remove substances inhibiting the catalytic action of the microbial biomass and the like even by the methods for removing impurities disclosed in these documents, and there was still room for improvement from the viewpoint of effectively hydrating acrylonitrile.

In addition, the methods disclosed in the documents still had room for improvement from the viewpoint of improving the quality of acrylamide and an acrylamide-based polymer.
[Patent Document 1] Japanese Patent Laid-Open Publication No. H11-123098
[Patent Document 2] International Publication Pamphlet WO 2004/090148

### [DISCLOSURE OF THE INVENTION]

### [PROBLEMS TO BE SOLVED BY THE INVENTION]

An object of the present invention is to provide a method for more efficiently producing acrylamide with higher quality by a microbial catalyst containing a nitrile hydratase, and the like.

### [MEANS TO SOLVE THE PROBLEMS]

The present inventors studied the problems and have found that, when acrylamide is produced by hydrating acrylonitrile by a microbial biomass containing a nitrile hydratase or a processed product of the microbial biomass, the catalytic activity of nitrile hydratase may be maintained and acrylamide with high quality may be obtained, if the reaction is performed in the presence of a compound having an active methylene group in the same molecule and/or a salt of the compound, and thus accomplished the present invention.

That is, the method for producing acrylamide of the present invention is characterized in that acrylonitrile is hydrated by a microbial biomass containing a nitrile hydratase or a processed product of the microbial biomass in the presence of a compound having at least an active methylene group in the same molecule and/or at least a salt of the compound.

As the compound having an active methylene group in the molecule, there may be preferably mentioned at least one compound selected from the group consisting of dimedone, barbituric acid and hydantoin.

In addition, as the salt of the compound having an active methylene group in the molecule, there may be preferably mentioned a salt of at least one compound selected from the group consisting of dimedone, barbituric acid and hydantoin.

### [EFFECT OF THE INVENTION]

According to the present invention, acrylamide with higher quality may be produced more efficiently by a microbial catalyst containing a nitrile hydratase, and the like.

### [BEST MODE FOR CARRYING OUT THE INVENTION]

Hereinafter, the present invention is explained in detail.

Firstly, there is explained a raw material used for a method of producing the acrylamide of the present invention.

### [Acrylonitrile]

The acrylonitrile used in the present invention is not particularly limited.

### [Microbial Biomass containing Nitrile Hydratase, and the like]

In the present invention, the acrylamide of the present invention may be obtained by hydrating the acrylonitrile used as a raw material by using as a catalyst a microbial biomass containing a nitrile hydratase or a processed product of the microbial biomass and the like.

In the present invention, the nitrile hydratase is referred to as an enzyme having capability of hydrolyzing a nitrile compound to produce a corresponding amide compound. Here, the microorganism containing a nitrile hydratase is not particularly limited as long as it produces a nitrile hydratase having capability of hydrolyzing a nitrile compound to produce a corresponding amide compound and maintains a nitrile hydratase activity in an aqueous solution of acrylamide.

Specifically, preferred examples of the microorganism include microorganisms belonging to Nocardia, Corynebacterium, Bacillus, thermophilic Bacillus, Pseudomonas, Micrococcus, Rhodococcus represented by the rhodochrous species, Acinetobacter, Xanthobacter, Streptomyces, Rhizobium, Klebsiella, Enterobacter, Erwinia, Aeromonas, Citrobacter, Achromobacter, Agrobacterium or Pseudonocardia represented by the thermophila species.

Further, the microorganism referred in the present invention also includes a transformant obtained by expressing a nitrile hydratase gene cloned from the microorganism in an arbitrary host. The arbitrary host referred herein includes, but not particularly limited to, Escherichia coli as a representative example as in the case of Examples described later, Bacillus such as Bacillus subtilis and the like and other microbial strains such as yeasts, Actinomyces and the like. An example of such includes MT-10822 (the strain has been deposited at National Institute of Bioscience and Human Technology, Agency of Industrial Science and Technology, Ministry of International Trade and Industry, 1-1, Higashi 1-chome, Tsukuba-shi, Ibaraki-ken, Japan (currently, International Patent Organism Depositary of National Institute of Advanced Industrial Science and Technology, Central 6, Higashi 1-1-1, Tusukuba-shi, Ibaraki-ken, Japan) on February 7th, 1996, under an accession number FERM BP-5785, under the terms of the Budapest Treaty on the International Recognition of the Deposit of Microorganisms for the Purposes of Patent Procedure). The microorganism referred in the present invention also includes a transformant obtained by expressing a mutant nitrile hydratase that has been further improved in acrylamide resistance, acrylonitrile resistance and temperature resistance by replacing, deleting, eliminating or inserting one or two or more of constituent amino acids of the enzyme with other amino acids by using recombinant DNA technology.

In producing an amide compound by using the microorganism as mentioned above, the microbial biomass or a processed product of the microbial biomass is generally used. The microbial biomass may be prepared by using a general method which is well known in the fields of molecular biology, bioengineering and genetic engineering. For example, there may be mentioned a method in which the microorganism is planted in a typical liquid culture medium such as an LB medium, an M9 medium and the like and then is grown at an appropriate culture temperature (which is generally from 20 to 50°C and may be 50°C or higher in the case of a thermophilic bacterium) , and obtained by separating and recovering from the culture liquid using a centrifugal separation.

In addition, the processed product of the microbial biomass in the present invention denotes an extract and a trituration product of the microbial biomass; a post-separated product obtained by separating and purifying a nitrile hydratase active fraction of the extract and the trituration product; and an immobilized product obtained by immobilizing the microbial biomass, or the extract, the trituration product or the post-separated product of the microbial biomass using an appropriate carrier, and the like, and these correspond to the processed product of the microbial biomass of the present invention as long as they have a nitrile hydratase activity. These may be used solely as a single species, or two or more different species thereof may be used simultaneously or alternately.

### [Compound having Active Methylene Group and the like]

In the present invention, in producing acrylamide by hydrating acrylonitrile by a microbial biomass containing a nitrile hydratase, and the like, the reaction is carried out in the presence of a compound having an active methylene group and/or its salt.

The active methylene group contained in the compounds is referred to as, for example, a methylene group having a general formula of X-CH₂-Y in which at least either X or Y, which is adjacent to the methylene group, is an electron attractive group such as NO₂, CN, COR, COAr, CONHR, CONHAr, COOR, COOH, SO₂, S, Ar, quaternary pyridinium and the like, as described in Organic Reactions Vol. 15 (1967), PP 222 - 223, John Wiley & Sons, Inc., provided that in the formula, R represents an alkyl group and Ar represents an aryl group.

In addition, in the present invention, there may be used a salt, for example, potassium salt, sodium salt, magnesium salt, calcium salt and the like of these compounds.

The compound having the active methylene group includes malonic acid, malonic acid monoester, malonic acid amide, cyanoacetic acid, cyanoacetic acid amide, acetoacetic acid, acetoaldehyde sulfonic acid, acetone sulfonic acid, sulfoacetic acid, sulfoacetic acid ester, sulfoacetic acid amide, dimedone, barbituric acid, hydantoin and the like.

Of these compounds or salts, preferred are dimedone, barbituric acid, hydantoin and a salt thereof because of their effect and easy availability, and more preferred are barbituric acid and its salt because of their effect.

In addition, in the present invention, the compounds may be used in combination with the salts. Further, the compounds may be used alone or in combination with two or more kinds, and the salts may used alone or in combination with two or more kinds.

The amount of these compounds and salts present is not particularly limited. However, in order that the resulting acrylamide is excellent in quality and the load in the purification process of acrylamide is not too large, the amount of the compounds present is typically in the range of 10 to 10, 000 ppm by weight and preferably in the range of 50 to 5, 000 ppm by weight, relative to the total amount of the reaction solution.

As a method of allowing these compounds to be present, there may be mentioned a method of adding the compounds after dissolving in an aqueous medium used in the reaction described later or the raw material acrylonitrile, a method of dissolving the compounds in a small amount of water or pouring the compounds directly in the reactor or the circulation system of the reaction solution, or the like.

### [Reaction Conditions]

In the present invention, acrylonitrile is typically hydrated in an aqueous medium by the microbial biomass and the like. Here, the aqueous medium in the present invention refers to water or an aqueous solution dissolving a buffer such as a phosphate and the like, an inorganic salt such as a sulfate, carbonate and the like, a hydroxide of alkali metal, an amide compound or the like at a suitable concentration.

In the present invention, the concentration of acrylonitrile in the aqueous medium is the saturated concentration or higher of the nitrile compound at the start of the reaction. The upper limit of the concentration is not particularly limited, but when an overly excessive amount of the nitrile compound is supplied, it is required to use a large amount of a catalyst and a reactor having an excessively large volume for completing the reaction, an excessively large heat exchanger for removing heat, and the like, causing economic burden with respect to equipment. For this reason, as the supplied concentration of acrylonitrile, acrylonitrile is preferably supplied such that, when all the acrylonitrile is converted to the corresponding acrylamide, the theoretical concentration of product solution is 40 to 80% by weight in the case of acrylamide, and more specifically, 0.4 to 1. 5 parts by weight of acrylonitrile based on 1.0 part by weight of water.

In addition, although the reaction time in the reaction depends on the conditions such as the amount of a catalyst used, the temperature and the like, when the reaction is carried out by using multiple reactors, the reaction time is usually in the range of 1 to 80 hours per one reactor and preferably in the range of 2 to 40 hours per one reactor.

The amount of the catalyst to be used varies depending on the reaction conditions, the type and form of the catalyst, and is usually 10 to 50000 ppm and preferably 50 to 30000 ppm of a dry microbial biomass with respect to the weight of the reaction solution.

In addition, the hydration reaction is generally carried out under normal pressure or pressure near normal pressure and may be carried out under increased pressure in order to increase the solubility of the nitrile compound in the aqueous medium. Further, the reaction temperature is not particularly limited as long as it is the freezing point or higher of the aqueous medium , and the reaction is preferably carried out in the range from 0 to 50°C and more preferably from 10 to 40°C. Furthermore, the reaction may also be carried out in a slurry state in which products are crystallized in the reaction solution. In addition, the pH of the reaction solution during the hydration reaction is not particularly limited as long as the nitrile hydratase activity is maintained, and is preferably in the range of pH 6 to 10 and more preferably in the range of pH 7 to 9.

### [Method for Purifying Acrylamide]

As a method for purifying acrylamide obtained in the present invention by removing a compound having an active methylene group and the like, and a reaction product of the compound and the like with impurities, there may be mentioned, for example, a method of bringing into contact with an anion exchange resin. The anion exchange resin is not particularly limited as long as it may remove these compounds and there may be preferably used a weakly basic or a medium basic or a strongly basic anion exchange resin.

AS the anion exchange anion resin which may be used in the present invention, there may be mentioned a macroporous-type weakly basic resin such as Lewatit MP62 (trade name, produced by LANXESS Deutschland GmbH), Diaion WA20 (trade name, produced by Mitsubishi Chemical Corporation), Dowex 66 (trade name, produced by Dow Chemical Company) and the like; a gel-type weakly basic resin such as Lewatit OC1059 (trade name, produced by LANXESS Deutschland GmbH) ; a gel-type medium basic resin such as Lewatit MP64 (trade name, produced by LANXESS Deutschland GmbH) or Amberlite IRA68 (trade name, produced by Organo Corporation) or the like; a macroporous-type medium basic resin such as Dowex WRG2 (trade name, produced by Dow Chemical Company) and the like; macroporous-type strongly basic resin such as Lewatit MP500 (trade name, produced by LANXESS Deutschland GmbH) ; a gel-type strongly basic resin such as Amberlite IRA400 (trade name, produced by Organo Corporation) and the like; and the like.

These commercially available anion exchange resins may be used after sufficiently washing with water, but they may be preferably used after pretreating with a dilute alkaline solution and then sufficiently washing with water.

These resins may be continuously contacted with an acrylamide solution to purify as a fixed layer such as a filling layer, and may be also used in a batch system. However, these resins are preferably used as a fixed layer because of the purification efficiency, ease of operation and the like. The acrylamide thus obtained is excellent in quality. When evaluating the acrylamide-based polymer obtained by homopolymerizing the acrylamide or by copolymerizing the acrylamide with other monomer, the solubility in water is dramatically increased, and the polymer has a sufficiently high molecular weight. In addition, the polymer obtained is excellent in hue.

### [Production of Acrylamide-based Polymer]

The acrylamide-based polymer may be produced by homopolymerizing the acrylamide of the present invention or by copolymerizing the acrylamide with other monomer.

The other monomer copolymerizable with acrylamide includes an unsaturated carboxylic acid such as acrylic acid, methacrylic acid, itaconic acid, maleic acid, fumaric acid and the like, and a salt thereof;
vinylsulfonic acid, styrene sulfonic acid and acrylamidemethylpropane sulfonic acid, and a salt thereof;
an alkylaminoalkyl ester of (meth)acrylic acid such as N,N-dimethylaminoethylmethacrylate,
N,N-diethylaminoethylmethacrylate,
N,N-dimethylaminoethylacrylate and the like, and a quaternary ammonium derivative thereof;
N,N-dialkylaminoalkyl(meth)acrylamide such as N,N-dimethylaminopropylmethacrylamide,
N,N-dimethylaminopropylacrylamide and the like, and a quaternary ammonium derivative thereof;
a hydrophilic acrylamide such as acetone acrylamide, N,N-dimethylacrylamide, N,N-dimethylmethacrylamide, N-ethylmethacrylamide, N-ethylacrylamide, N,N-diethylacrylamide, N-propylacrylamide and the like;
N-acryloylpyrrolidine, N-acryloylpiperidine and N-acryloylmorpholine;
hydroxyethylmethacrylate, hydroxyethylacrylate, hydroxypropylmethacrylate and hydroxypropylacrylate;
methoxypolyethyleneglycol(meth)acrylate and N-vinyl-2-pyrrolidone;
an N-alkyl(meth)acrylamide derivative such as N,N-di-n-propylacrylamide, N-n-butylacrylamide, N-n-hexylacrylamide, N-n-hexylmethacrylamide, N-n-octylacrylamide, N-n-octylmethacrylamide, N-tert-octylacrylamide, N-dodecylacrylamide, N-n-dodecylmethacrylamide and the like;
an N-(ω-glycidoxyalkyl) (meth)acrylamide derivative such as N,N-diglycidylacrylamide, N,N-diglycidylmethacrylamide, N-(4-glycidoxybutyl)acrylamide, N-(4-glycidoxybutyl)methacrylamide, N-(5-glycidoxypentyl)acrylamide, N-(6-glycidoxyhexyl)acrylamide and the like;
a (meth)acrylate derivative such as methyl(meth)acrylate, ethyl(meth)acrylate, butyl(meth)acrylate, lauryl(meth)acrylate, 2-ethylhexyl(meth)acrylate, glycidyl(meth)acrylate and the like;
olefins such as acrylonitrile, methacrylonitrile, vinyl acetate, vinyl chloride, vinylidene chloride, ethylene, propylene, butene and the like; styrene, α-methylstyrene, butadiene, isoprene, methacrylamide and the like.

These monomers may be used alone or two or more kinds in combination.

The mixing ratio when acrylamide is copolymerized with the other monomer is not particularly limited, but is typically 100 mol or less and preferably 50 mol or less, relative to 100 mol of acrylamide.

The polymerization method of these monomers includes, for example, an aqueous solution polymerization, an emulsion polymerization and the like.

Among these, in the case of the aqueous solution polymerization, the total concentration of acrylamide and other monomer which is added as needed is typically 5 to 90% by weight.

As a polymerization initiator, for example, a radical polymerization initiator may be used.

As the radical polymerization initiator, there may be mentioned a peroxide such as potassium persulfate, ammonium persulfate, hydrogen peroxide, benzoyl peroxide and the like; an azo-based free radical initiator such as azobisisobutyronitrile, 2,2'-azobis(4-amidinopropane) dihydrochloride, sodium 4,4'-azobis(4-cyanovalerate) and the like; and a so-called redox catalysts using in combination the peroxides mentioned above and a reducing agent such as sodium bisulfite, triethanolamine, ammonium ferrous sulfate and the like, and dimethylaminopropionitrile.

The polymerization initiators mentioned above may be used alone or two or more kinds in combination. The amount of the polymerization initiator is typically 0.001 to 5% by weight relative to the total amount of the monomer.

The polymerization temperature is usually in the range of -10 to 120°C and preferably in the range of 0 to 90°C. In addition, the polymerization temperature is not required to be always kept constant but may be changed accordingly with the progress of the polymerization. Since the polymerization heat is usually generated with the progress of the polymerization leading to an increase in the polymerization temperature, cooling may be provided as needed.

The atmosphere during the polymerization is not particularly limited but the polymerization is preferably carried out, for example, under an inert gas atmosphere such as a nitrogen gas and the like, from the viewpoint of promoting the polymerization.

The polymerization time is not particularly limited but usually in the range of 1 to 20 hours.

In addition, the pH of the solution during the polymerization is not particularly limited but the polymerization may be carried out by adjusting the pH as needed. In this case, examples of useful pH adjusting agent include an alkali such as sodium hydroxide, potassium hydroxide, ammonia and the like; a mineral acid such as phosphoric acid, sulfuric acid, hydrochloric acid and the like; an organic acid such as formic acid, acetic acid and the like; and others.

The molecular weight of the polymer obtained from the present invention is not particularly limited but is typically in the range of 100,000 to 50,000,000 and preferably in the range of 500,000 to 30,000,000.

The acrylamide-based polymer thus obtained has a dramatically increased solubility in water, has a sufficiently high molecular weight and is furthermore excellent in hue because the acrylamide obtained by the present invention has excellent quality. Therefore, the acrylamide-based polymer may be preferably used as a flocculant, an additive for manufacturing paper, an oil recovery agent and the like.

### [EXAMPLES]

Hereinafter, the present invention will be explained in more detail with reference to the Examples, but the present invention is not limited by these Examples.

### [Example 1]

### (Addition of Barbituric Acid in Hydration Reaction)

### [Culture of Microbial Biomass containing Nitrile Hydratase]

In accordance with the method described in Japanese Patent Laid-Open Publication No. 2001-340091, No. 3 cloned microbial biomass was obtained, which was cultured in the same manner as in Example 1 to obtain the wet microbial biomass containing a nitrile hydratase.

### [Production of Acrylamide]

There were prepared a 1-L glass flask equipped with a stirrer as the first reactor and a Teflon (trademark) tube with an inside diameter of 5 mm and a length of 40 m as the second reactor. To the first reactor was charged 400 g of water in advance.

The wet microbial biomass obtained by the culture method was suspended in purified water so that the content was 12% by weight. The suspension was continuously fed into the first reactor under stirring at a rate of 11 g/h. Acrylonitrile containing 30 ppm by weight of oxazole and 2 ppm by weight of acrolein was continuously fed at a rate of 31 g/h, and an aqueous solution of barbituric acid prepared by adding barbituric acid in purified water so that the content is 550 ppm by weight was continuously fed at a rate of 38 g/h. Further, an aqueous solution of 0.1 M-NaOH was continuously fed to adjust the pH of the reaction solution at 7.5 to 8.5. In addition, the reaction solution was continuously taken out from the first reactor at a rate of 80 g/h so that the liquid level of the first reactor was maintained constant. Furthermore, the liquid taken out was continuously fed into the second reactor at a rate of 80 g/h and the reaction was further promoted in the second reactor.

Both the first and second reactors were immersed in a water bath at a temperature of 10 to 20°C to control the liquid temperature inside each reactor at 15°C.

The reaction solution in each of the reactors was sampled on the second day from the start of the operation and analyzed by HPLC. As the result, the conversion from acrylonitrile to acrylamide at the outlet of the first reactor was 90%, and the concentration of acrylonitrile in the acrylamide at the outlet of the second reactor was at a detection limit or less (100 ppm by weight or less).

The resulting reaction solution was treated with activated carbon under acidic conditions (pH 5) to remove the wet microbial biomass. In addition, 200 ml of Lewatit MP500 which has been changed to the OH-type using an aqueous solution of 1 M-NaOH in advance was filled in a glass column. The resulting reaction solution was passed through the column at a flow rate of SV=1 (l/h) to remove impurities. Finally, the resulting reaction solution was neutralized to pH of 7 with 1 M-sulfuric acid to obtain an aqueous solution of 50% by weight of acrylamide.

### (Evaluation of Polymer Quality)

### [Example 2]

Water was added to the acrylamide solution obtained in Example 1 to obtain an aqueous solution of 20% by weight of acrylamide. In a 1-L polyethylene vessel was placed 500 g of the aqueous solution of 20% by weight of acrylamide and the dissolved oxygen in the solution was removed by passing nitrogen while maintaining the temperature at 18°C, and the polyethylene vessel was immediately placed in a heat-insulating block made of expanded polystyrene foam.

Subsequently, 200×10⁻⁶ mpm (a molar ratio to acrylamide) of sodium 4,4'-azobis-4-cyanovalerate, 200×10⁻⁶ mpm of dimethylaminopropionitrile and 80×10⁻⁶ mpm of ammonium persulfate were each dissolved in a small amount of water, and then the resultant reagents were promptly poured into the 1-L polyethylene vessel in this order. To these reagents, nitrogen gas was purged in advance, and during, before and after the pouring of these reagents, a small amount of nitrogen gas was purged into the polyethylene vessel to prevent an oxygen gas from being mixed into the solution.

After an induction period of several minutes subsequent to the pouring of the reagents, the feeding of nitrogen gas was stopped because the temperature inside of the polyethylene vessel was observed to rise. When the polyethylene vessel was kept as it is in the heat-insulating block for approximately 100 minutes, the temperature inside of the polyethylene vessel reached approximately 70°C. Consequently, the polyethylene vessel was taken out from the heat-insulating block and immersed in water at 97°C for 2 hours to further promote the polymerization reaction. Thereafter, the polyethylene vessel was immersed in cold water to cool and quench the polymerization reaction.

The water-containing gel of the acrylamide polymer thus obtained was taken out from the polyethylene vessel, divided into small pieces, and ground through a mincer. The ground water-containing gel of the acrylamide polymer was dried with hot air at 100°C for 2 hours and further ground by a high-speed rotary blade grinder to obtain an acrylamide polymer in a state of a dry powder.
The resulting acrylamide polymer in a state of a dry powder was sieved to collect the powder passing through 32 to 42 mesh. The collected acrylamide polymer was evaluated by the test method of the acrylamide polymer of Example 5 described later. The results are shown in Table 1.

### [Example 3]

### (Addition of Salt of Barbituric Acid in Hydration Reaction)

An aqueous solution of sodium barbiturate was prepared by adding an aqueous solution of 0.1 M-NaOH to the aqueous solution of barbituric acid so that the pH is 7.0.

There were prepared a 1-L glass flask equipped with a stirrer as the first reactor and a Teflon (trademark) tube with an inside diameter of 5 mm and a length of 40 m as the second reactor. To the first reactor was charged 400 g of water in advance.

The wet microbial biomass obtained by the culture method of Example 1 was suspended in purified water so that the content was 12% by weight. The suspension was continuously fed into the first reactor under stirring at a rate of 11 g/h. Acrylonitrile containing 30 ppm by weight of oxazole and 2 ppm by weight of acrolein was continuously fed at a rate of 31 g/h, and an aqueous solution of sodium barbiturate prepared by adding the sodium barbiturate solution in purified water so that the content is 550 ppm by weight equivalent of barbituric acid was continuously fed at a rate of 38 g/h. Further, an aqueous solution of 0.1 M-NaOH was continuously fed to adjust the pH of the reaction solution at 7.5 to 8.5. In addition, the reaction solution was continuously taken out from the first reactor at a rate of 80 g/h so that the liquid level of the first reactor was maintained constant. Furthermore, the liquid taken out was continuously fed into the second reactor at a rate of 80 g/h and the reaction was further promoted in the second reactor.

Both the first and second reactors were immersed in a water bath at a temperature of 10 to 20°C to control the liquid temperature inside each reactor at 15°C.

The reaction solution in each of the reactors was sampled on the second day from the start of the operation and analyzed by HPLC. As the result, the conversion from acrylonitrile to acrylamide at the outlet of the first reactor was 90%, and the concentration of acrylonitrile in acrylamide at the outlet of the second reactor was at a detection limit or less (100 ppm by weight or less).

The resulting reaction solution was treated with activated carbon under acidic conditions (pH 5) to remove the wet microbial biomass. In addition, 200 ml of Lewatit MP500 which has been changed to the OH-type using an aqueous solution of 1 M-NaOH in advance was filled in a glass column. The resulting reaction solution was passed through the column at a flow rate of SV=1 (l/h) to remove impurities. Finally, the resulting reaction solution was neutralized to pH of 7 with 1 M-sulfuric acid to obtain an aqueous solution of 50% by weight of acrylamide.

A sample of acrylamide polymer was obtained by performing a procedure by using this aqueous solution of acrylamide similar to that in Example 2. The resulting acrylamide polymer sample was evaluated by the test method of the acrylamide polymer of Example 5 described later. The results are shown in Table 1.

### [Example 4]

### (Addition of Hydantoin in Hydration Reaction)

The operation was carried out in the same manner as in Example 1 except for using hydantoin in place of barbituric acid.

The reaction solution in each of the reactors was sampled on the second day from the start of the operation and analyzed by HPLC. As the result, the conversion from acrylonitrile to acrylamide at the outlet of the first reactor was 91%, and the concentration of acrylonitrile in acrylamide at the outlet of the second reactor was at a detection limit or less (100 ppm by weight or less).

The resulting reaction solution was treated with activated carbon under acidic conditions (pH 5) to remove the wet microbial biomass. In addition, 200 ml of Lewatit MP500 which has been changed to the OH-type using an aqueous solution of 1 M-NaOH in advance was filled in a glass column. The resulting reaction solution was passed through the column at a flow rate of SV=1 (l/h) to remove impurities. Finally, the resulting reaction solution was neutralized to pH of 7 with 1 M-sulfuric acid to obtain an aqueous solution of 50% by weight of acrylamide.

A sample of acrylamide polymer was obtained by performing a procedure using this aqueous solution of acrylamide similar to that in Example 2. The collected acrylamide polymer in a state of a dry powder was sieved to collect the powder passing through 32 to 42 mesh. The resulting acrylamide polymer was evaluated by the test method of the acrylamide polymer of Example 5 described later. The results are shown in Table 1.

### [Comparative Example 1]

### (Compound having Active Methylene Group is not added in Hydration Reaction)

The wet microbial biomass obtained by the culture method of Example 1 was suspended in purified water so that the content was 12% by weight. The suspension was continuously fed into the first reactor under stirring at a rate of 11 g/h. Acrylonitrile containing 30 ppm by weight of oxazole and 2 ppm by weight of acrolein was continuously fed at a rate of 31 g/h, and purified water was continuously fed at a rate of 38 g/h. Further, an aqueous solution of 0.1 M-NaOH was continuously fed to adjust the pH of the reaction solution at 7.5 to 8.5. In addition, the reaction solution was continuously taken out from the first reactor at a rate of 80 g/h so that the liquid level of the first reactor was maintained constant. Furthermore, the liquid taken out was continuously fed into the second reactor at a rate of 80 g/h and the reaction was further promoted in the second reactor.

Both the first and second reactors were immersed in a water bath at a temperature of 10 to 20°C to control the liquid temperature inside each reactor at 15°C.

The reaction solution in each of the reactors was sampled on the second day from the start of the operation and analyzed by HPLC. As the result, the conversion from acrylonitrile to acrylamide at the outlet of the first reactor was 87%, and the concentration of acrylonitrile in acrylamide at the outlet of the second reactor was at a detection limit or less (100 ppm by weight or less).

The resulting reaction solution was treated with activated carbon under acidic conditions (pH 5) to remove the wet microbial biomass. In addition, 200 ml of Lewatit MP500 which has been changed to the OH-type using an aqueous solution of 1 M-NaOH in advance was filled in a glass column. The resulting reaction solution was passed through the column at a flow rate of SV=1 (l/h) to remove impurities. Finally, the resulting reaction solution was neutralized to pH of 7 with 1 M-sulfuric acid to obtain an aqueous solution of 50% by weight of acrylamide.

A sample of acrylamide polymer was obtained by performing a procedure using this aqueous solution of acrylamide similar to that in Example 2. The resulting acrylamide polymer in a state of a dry powder was sieved to collect the powder passing through 32 to 42 mesh. The collected acrylamide polymer was evaluated by the test method of the acrylamide polymer of Example 5 described later. The results are shown in Table 1.

### [Example 5]

### <Test Method of Acrylamide Polymer>

For the polymer sample obtained in the Examples 2 to 4. and Comparative Example 1, the evaluation of solubility in water, measurement of the standard viscosity and evaluation of hue were performed using the following methods.
Solubility in water: Into an 1-L beaker was placed 600 ml of water and 0. 66 g (net content: 0. 6 g) of the polymer sample was added while stirring at 25°C by using a stirring blade having a defined shape, followed by stirring at 400 rpm for 2 hours. The resulting solution was filtered through a 150-mesh metal wire screen. The solubility in water of the polymer sample was judged from the amount of insoluble matter and the filterability. That is to say, the evaluation was made as follows. Excellent: Completely dissolved; Good: Almost completely dissolved; Do: Insoluble matter was present but separated by filtration; and Poor: The passing of the filtrate was so slow that filtration of insoluble matter was practically impossible.
Standard viscosity: The filtrate obtained in the solubility test was an aqueous solution of polymer having a concentration of 0.1% by weight. To the aqueous solution of polymer was added sodium chloride with a concentration equivalent to 1 M. By using a BL-type viscometer equipped with a BL adapter, the viscosity (standard viscosity) of the resulting solution was measured at 25°C and a rotor revolution speed of 60 rpm. The standard viscosity obtained in this manner is commonly employed as a value correlated with the molecular weight.
Hue: For the hue of the polymer, the polymer powders were visually evaluated.
The evaluation results are shown in Table 1.

**[Table 1]**

| | Additive | Solubility of Polymer | Viscosity of Aqueous Solution of Polymer (mPa.s) | Hue of Polymer (Visual Observation) |
|---|---|---|---|---|
| Example 2 | Barbituric acid | Excellent | 5.5 | White |
| Example 3 | Sodium barbiturate | Excellent | 5.5 | White |
| Example 4 | Hydantoin | Good | 5.4 | White |
| Comparative Example 1 | No addition | Poor | No measurement done* | Light Yellow |

| | | | | |
|---|---|---|---|---|
| *) The viscosity of the filtrate was impossible to be measured because the passing of the filtrate was slow and the filtration was practically impossible. | | | | |

## Claims

1. A method for producing acrylamide by hydrating acrylonitrile by a microbial biomass containing a nitrile hydratase or a processed product of the microbial biomass in the presence of at least one compound having an active methylene group and/or at least one salt of the compound.

2. The method for producing acrylamide according to claim 1, wherein the compound having an active methylene group and/or the salt of the compound is at least one compound and/or its salt selected from the group consisting of dimedone, barbituric acid, hydantoin and/or a salt thereof.
